# EUROPEAN PATENT SPECIFICATION

(11) **EP 4 512 473 B1**
(45) Date of publication and mention of the grant of the patent: **24.06.2026**
(21) Application number: 24205125.8
(22) Date of filing: 03.08.2018
(51) Int. Cl.: A61K 31/44, A61P 25/00, A61P 25/24, A61P 25/16, A61P 25/14, A61P 25/28, A61P 25/18, A61P 25/30, A61P 25/08

(54) **VAFIDEMSTAT FOR TREATING BEHAVIOR ALTERATIONS**
VAFIDEMSTAT ZUR BEHANDLUNG VON VERHALTENSVERÄNDERUNGEN
VAFIDEMSTAT DANS LES PROCÉDÉS DE TRAITEMENT D'ALTÉRATIONS DE COMPORTEMENT

(30) Priority: 03.08.2017 EP 17382544; 03.08.2017 EP 17382545; 30.04.2018 EP 18382299
(43) Date of publication of application: 26.02.2025
(62) Divisional of application: 18748921.6
(73) Proprietor: Oryzon Genomics, S.A., 28014 Madrid (ES)
(72) Inventor: MAES, Tamara, 08940 Cornellà de Llobregat (ES); ROTLLANT POZO, David, 08940 Cornellà de Llobregat (ES); GRIÑÁN FERRE, Christian, 08940 Cornellà de Llobregat (ES); PALLÀS LLIBERIA, Mercè, 08940 Cornellà de Llobregat (ES); NADAL ALEMANY, Roser, 08940 Cornellà de Llobregat (ES); ARMARIO GARCÍA, Antonio, 08940 Cornellà de Llobregat (ES)
(74) Representative: Vossius & Partner Patentanwälte Rechtsanwälte mbB

(56) References cited:
- WO-A1-2012/013728
- CARLOS BUESA ET AL: "THE DUAL LSD1/MAOB INHIBITOR ORY2001 PREVENTS THE DEVELOPMENT OF THE MEMORY DEFICIT IN SAMP8 MICE THROUGH INDUCTION OF NEURONAL PLASTICITY AND REDUCTION OF NEUROINFLAMMATION", 1 July 2015 (2015-07-01), XP055438264, Retrieved from the Internet <URL:http://www.alzheimersanddementia.com/article/S1552-5260(15)02849-6/pdf> [retrieved on 20180104]
- I N ORYZON ET AL: "Oryzon - a global leader in epigenetics", MARKETWIRED, February 2016 (2016-02-01), Toronto, XP055438247, Retrieved from the Internet <URL:https://www.oryzon.com/sites/default/files/20160208 BioCEO ORY Presentation.pdf> [retrieved on 20180104]
- MAES TAMARA ET AL: "ORY-2001: AN EPIGENETIC DRUG FOR THE TREATMENT OF COGNITION DEFECTS IN ALZHEIMER'S DISEASE AND OTHER NEURODEGENERATIVE DISORDERS", ALZHEIMER'S & DEMENTIA: THE JOURNAL OF THE ALZHEIMER'SASSOCIATION, vol. 12, no. 7, 27 July 2016 (2016-07-27), XP029771061, ISSN: 1552-5260, DOI: 10.1016/J.JALZ.2016.07.149
- TAMARA MAES ET AL: "KDM1 histone lysine demethylases as targets for treatments of oncological and neurodegenerative disease", EPIGENOMICS, vol. 7, no. 4, 1 June 2015 (2015-06-01), United Kingdom, pages 609 - 626, XP055438065, ISSN: 1750-1911, DOI: 10.2217/epi.15.9
- CYRIL JAYAKUMAR PETER ET AL: "Balancing histone methylation activities in psychiatric disorders", TRENDS IN MOLECULAR MEDICINE, vol. 17, no. 7, July 2011 (2011-07-01), pages 372 - 379, XP028238948, ISSN: 1471-4914, [retrieved on 20110216], DOI: 10.1016/J.MOLMED.2011.02.003
- R. BULLOCK ET AL: "2695. Vafidemstat reduces aggressiveness in three different psychiatric disorders. Final data from the REIMAGINE trial", 4 July 2020 (2020-07-04), pages 1, XP055746209, Retrieved from the Internet <URL:https://www.oryzon.com/sites/default/files/20200706_EPA2020_poster.pdf> [retrieved on 20201102]

## Description

### FIELD

The present invention relates to a KDM1A inhibitor, which is 5-((((1R,2S)-2-(4-(benzyloxy)phenyl)cyclopropyl)amino)methyl)-1,3,4-oxadiazol-2-amine or a pharmaceutically acceptable salt or solvate thereof, for use in the treatment of a behavior alteration which is social withdrawal or aggressiveness, wherein said social withdrawal or said aggressiveness is associated with a disease.

### BACKGROUND

Behavior alterations, such as for example social withdrawal or aggressive behavior, are highly prevalent in today's society and are viewed by clinicians as a medical condition in its own right. Still, the treatment of behavioral alterations remains a medical challenge. There are currently no approved drugs that specifically aim at the treatment of social withdrawal, aggressive behavior or other behavioral alterations. In addition, many of the existing medications that are being used to treat behavior alterations can cause severe side effects; for example, many antispychotic drugs (also known as neuroleptics or major tranquilizers), which are being used to treat aggressive behavior and other behavior alterations, cause sedation.

Thus, there is a strong and unmet need for new and/or improved drugs for treating behavior alterations, particularly drugs acting via novel mechanisms of action that allow to specifically treat behavior alterations and/or exhibiting a more favorable side effect profile than current treatments. The present invention addresses these and other needs.

### SUMMARY OF THE INVENTION

The present invention provides a KDM1A inhibitor which is 5-((((1R,2S)-2-(4-(benzyloxy)phenyl)cyclopropyl)amino)methyl)-1,3,4-oxadiazol-2-amine, or a pharmaceutically acceptable salt or solvate thereof, for use in the treatment of a behavior alteration, wherein the behavior alteration is social withdrawal or aggressiveness, wherein said social withdrawal or said aggressiveness is associated with a disease.

The present invention further provides a pharmaceutical composition for use in the treatment of a behavior alteration, wherein the behavior alteration is social withdrawal or aggressiveness, wherein said social withdrawal or said aggressiveness is associated with a disease, and wherein the pharmaceutical composition comprises a KDM1A inhibitor which is 5-((((1R,2S)-2-(4-(benzyloxy)phenyl)cyclopropyl)amino)methyl)-1,3,4-oxadiazol-2-amine, or a pharmaceutically acceptable salt or solvate thereof, and one or more pharmaceutically acceptable excipients or carriers.

### BRIEF DESCRIPTION OF THE DRAWINGS

Figure 1 shows the effect of treatment with the KDM1A inhibitor Compound 1 (as defined below and in Example 1) on aggressive behavior in the resident intruder test in male SAMP8 mice, as assessed by the total number of attacks, as described in more detail in Example 3. Means and Standard error of the Mean (SEM) are represented. * p < 0.05; ** p < 0.01.
Figure 2 shows the effect of treatment with Compound 1 on aggressive behavior in the resident intruder test in male SAMP8 mice, as assessed by the number of clinch attacks, as described in more detail in Example 3. Means and SEM are represented. ** p < 0.01; *** p < 0.001.
Figure 3 shows the effect of Compound 1 treatment on social avoidance in the resident intruder test in the rat isolation model, as assessed by the time without social interaction (measured in seconds), as described in more detail in Example 4. Means and SEM are represented. * p < 0.05; ** p < 0.01.
Figure 4 shows the effect of Compound 1 on social avoidance in the resident intruder test in the rat isolation model, as assessed by the number of evitations, as described in more detail in Example 4. Means and SEM are represented. * p < 0.05; *** p < 0.001.
Figure 5 shows the effect of Compound 1 on social interaction behaviour in the three chamber test (TCT) , as assessed by the time spent in each of the Object chamber and Mice chamber (measured in seconds), as described in more detail in Example 5. Means and SEM are represented. *** p < 0.001.
Figure 6 shows the effect of Compound 1 on social interaction behaviour in the TCT, as assessed by the time spent directly exploring the novel mice (measured in seconds), as described in more detail in Example 5. Means and SEM are represented. *** p < 0.001.

### DETAILED DESCRIPTION OF THE INVENTION

The present invention is based on the finding that KDM1A inhibitors are useful as therapeutic agents for the treatment of behavior alterations, as explained in more detail herein below and illustrated in the Examples.

Accordingly, the present invention provides a KDM1A inhibitor which is 5-((((1R,2S)-2-(4-(benzyloxy)phenyl)cyclopropyl)amino)methyl)-1,3,4-oxadiazol-2-amine, or a pharmaceutically acceptable salt or solvate thereof, for use in the treatment of a behavior alteration, wherein the behavior alteration is social withdrawal or aggressiveness, wherein said social withdrawal or said aggressiveness is associated with a disease.

The present invention likewise provides a KDM1A inhibitor which is 5-((((1R,2S)-2-(4-(benzyloxy)phenyl)cyclopropyl)amino)methyl)-1,3,4-oxadiazol-2-amine, or a pharmaceutically acceptable salt or solvate thereof, for use in treating a behavior alteration in a patient (preferably a human), wherein the behavior alteration is social withdrawal or aggressiveness, wherein said social withdrawal or said aggressiveness is associated with a disease, and wherein said use comprises administering to the patient a therapeutically effective amount of said KDM1A inhibitor.

In accordance with the present invention, a "behavior alteration" relates, in particular, to an alteration, disturbance, dysfunction, aberration, disorder or the like affecting a subject's behavior, including for example and without limitation, behavior alterations induced by or associated with genetic or epigenetic variations, behavior alterations associated with a disease, behavior alterations induced by drug therapy, behavior alterations induced by acute and/or chronic drug of abuse consumption, or behavior alterations induced by adverse social environment (for example: abandonment or neglect during childhood, traumatic experiences like warfare or sexual assault during adulthood) among others. Behavior alterations in accordance with the present invention do not include alterations in cognitive function (e.g. memory impairment) or mood (e.g. anxiety).

A social behavior alteration relates to an alteration, disturbance, dysfunction, aberration, disorder or the like affecting a subject's social behavior for whatever cause, including for example and without limitation, alterations in social behavior (e.g. social interaction alterations or aggressiveness) induced by or associated with genetic or epigenetic variations, alterations in social behavior (e.g. social interaction alterations or aggressiveness) associated with a disease, alterations in social behavior (e.g. social interaction alterations or aggressiveness) induced by drug therapy, alterations in social behavior (e.g. social interaction alterations or aggressiveness) induced by acute and/or chronic drug of abuse consumption, or alterations in social behavior (e.g. social interaction alterations or aggressiveness) induced by adverse social environment (for example: abandonment or neglect during childhood, traumatic experiences like warfare or sexual assault during adulthood), among others. Examples of social behavior alterations include, without limitation, social withdrawal, aggressiveness, or apathy, among others.

In accordance with the present invention, the behavior alteration to be treated is social withdrawal or aggressiveness, wherein said social withdrawal or said aggressiveness is associated with a disease.

The present invention thus relates to a KDM1A inhibitor which is 5-((((1R,2S)-2-(4-(benzyloxy)phenyl)cyclopropyl)amino)methyl)-1,3,4-oxadiazol-2-amine, or a pharmaceutically acceptable salt or solvate thereof, for use in the treatment of a social behavior disturbance, a social behavior dysfunction, a social behavior aberration, or a social behavior disorder, wherein the social behavior disturbance, the social behavior dysfunction, the social behavior aberration, or the social behavior disorder is social withdrawal or aggressiveness, wherein said social withdrawal or said aggressiveness is associated with a disease.

"Social withdrawal" in accordance with the present invention in particular relates to an abnormal, pathological or inappropriate lack of social interaction and/or reduced extent of social interaction (including social avoidance) for members of a social species like humans, particularly a condition in which an individual is retreating from society and interindividual relationships through the consistent (across situations and over time) display of solitary behavior in the presence of others, often accompanied by indifference or aloofment. In this regard, social withdrawal (which can also be referred to as passive withdrawal) is seen to arise from internal factors, with the individual opting, for some reason or another, not to interact with others. Social withdrawal according to the invention does not include active social isolation, used to denote a lack of social interaction attributed to external factors, for example the process whereby an individual stays alone because his/her peers do not wish to interact with him/her (that is, the individual is isolated by others). The social withdrawal to be treated in accordance with the present invention is associated with a disease (including e.g. autism spectrum disorder (ASD, such as, e.g., autism or Asperger's syndrome), avoidant personality disorder (AvPD), schizophrenia (including, e.g., a schizotypal and/or delusional disorder), a mood disorder (including, e.g., major depressive disorder; dysthymic disorder; or bipolar disorder), drug addiction, post-traumatic stress disorder (PTSD), dementia (including e.g. Alzheimer's disease), paranoid personality disorder, depressive personality disorder, schizoaffective disorder, traumatic brain injury (TBI), or an eating disorder (including e.g. bulimia nervosa)).

In accordance with the present invention, "aggressiveness" refers, in particular, to any kind of abnormal, pathological or inappropriate aggressive or violent behavior, hostility or agitation, for example physical or verbal, including interpersonal aggressiveness (i.e. towards other subjects) and/or intrapersonal aggressiveness (i.e. self-aggressiveness). The aggressiveness to be treated in accordance with the invention is associated with a disease (including e.g. Alzheimer's disease (AD), Huntington's disease (HD), dementia Lewy Body (DLB), Parkinson's disease (PD), schizophrenia (SZ), bipolar disorder (BPD), depression (DS), traumatic brain injury (TBI), REM sleep behaviour disorder (RBD), dementia, Dentatorubral-pallidoluysian atrophy (DRPLA), Tourette Syndrome (GTS), a conduct disorder (including e.g., unsocialized conduct disorder, socialized conduct disorder, or oppositional defiant disorder), drug addiction, a stress-related disorder (including, e.g., post-traumatic stress disorder), autism spectrum disorder (ASD), borderline personality disorder, or adult attention deficit hyperactivity disorder).

In some embodiments, the behavior alteration is social withdrawal associated with a disease. In some embodiments, said disease is a CNS disease. In some embodiments, said CNS disease is an autism spectrum disorder (ASD, such as, e.g., autism or Asperger's syndrome), avoidant personality disorder (AvPD), schizophrenia (including, e.g., a schizotypal and/or delusional disorder), a mood disorder (including, e.g., major depressive disorder; dysthymic disorder; or bipolar disorder), drug addiction, post-traumatic stress disorder (PTSD), dementia (including e.g. Alzheimer's disease), paranoid personality disorder, depressive personality disorder, schizoaffective disorder, TBI, or an eating disorder (including e.g. bulimia nervosa).

In some embodiments, the behavior alteration is aggressiveness associated with a disease. In some embodiments, said disease is a CNS disease. In some embodiments, said CNS disease is AD, HD, DLB, PD, SZ, BPD, DS, TBI, RBD, dementia, DRPLA, GTS, a conduct disorder (e.g., unsocialized conduct disorder, socialized conduct disorder, or oppositional defiant disorder), drug addiction, a stress-related disorder (including, e.g., post-traumatic stress disorder), ASD, borderline personality disorder or adult attention deficit hyperactivity disorder. In a preferred embodiment, the disease is AD. However, the disease may also be different from AD. For example, the disease may be HD, DLB, PD, SZ, BPD, DS, TBI, RBD, dementia, DRPLA, GTS, a conduct disorder (e.g., unsocialized conduct disorder, socialized conduct disorder, or oppositional defiant disorder), drug addiction, a stress-related disorder (including, e.g., post-traumatic stress disorder), ASD, borderline personality disorder or adult attention deficit hyperactivity disorder.

As explained above, the KDM1A inhibitor for use in accordance with the invention is the compound 5-((((1R,2S)-2-(4-(benzyloxy)phenyl)cyclopropyl)amino)methyl)-1,3,4-oxadiazol-2-amine, or a pharmaceutically acceptable salt or solvate thereof, and it is particularly preferred that the KDM1A inhibitor is the compound 5-((((1R,2S)-2-(4-(benzyloxy)phenyl)cyclopropyl)amino)methyl)-1,3,4-oxadiazol-2-amine (in non-salt form). This compound is also designated herein (including in the Examples and Figures) as Compound 1 or Comp. 1. The names "5-((((1R,2S)-2-(4-(benzyloxy)phenyl)cyclopropyl)amino)methyl)-1,3,4-oxadiazol-2-amine", "Compound 1" or "Comp. 1" are used herein interchangeably.

Accordingly, the present invention provides 5-((((1R,2S)-2-(4-(benzyloxy)phenyl)cyclopropyl)amino)methyl)-1,3,4-oxadiazol-2-amine, or a pharmaceutically acceptable salt or solvate thereof, for use in the treatment of a behavior alteration, wherein the behavior alteration is social withdrawal or aggressiveness, wherein said social withdrawal or said aggressiveness is associated with a disease.

In some embodiments, the behavior alteration is social withdrawal which is associated with a disease.

In some embodiments, the behavior alteration is aggressiveness which is associated with a disease.

Preferably, the KDM1A inhibitor for use in accordance with the invention, i.e. the compound 5-((((1R,2S)-2-(4-(benzyloxy)phenyl)cyclopropyl)amino)methyl)-1,3,4-oxadiazol-2-amine (or a pharmaceutically acceptable salt or solvate thereof), is administered orally. Exemplary formulations which can be administered via peroral ingestion are described in more detail further below.

As explained above, the present invention provides the compound 5-((((1R,2S)-2-(4-(benzyloxy)phenyl)cyclopropyl)amino)methyl)-1,3,4-oxadiazol-2-amine, or a pharmaceutically acceptable salt or solvate of said compound, for use in the treatment of a behavior alteration, wherein the behavior alteration is social withdrawal or aggressiveness, wherein said social withdrawal or said aggressiveness is associated with a disease. Accordingly, the invention relates to the compound 5-((((1R,2S)-2-(4-(benzyloxy)phenyl)cyclopropyl)amino)methyl)-1,3,4-oxadiazol-2-amine as a free base (in non-salt form) for use in the treatment of a behavior alteration which is social withdrawal or aggressiveness, wherein said social withdrawal or said aggressiveness is associated with a disease. Furthermore, the invention also relates to a pharmaceutically acceptable salt or solvate of 5-((((1R,2S)-2-(4-(benzyloxy)phenyl)cyclopropyl)amino)methyl)-1,3,4-oxadiazol-2-amine for use in the treatment of a behavior alteration which is social withdrawal or aggressiveness, wherein said social withdrawal or said aggressiveness is associated with a disease.

The findings underlying the present invention (which has been described herein above and is defined in the claims) are further explained in the following.

As also illustrated in the Examples, it has surprisingly been found in the context of the present invention that KDM1A inhibitors such as, e.g., Compound 1 provide potent therapeutic effects in animal models of (human) behavior alterations. In particular, the beneficial effects of KDM1A inhibitors have been observed on different types of behavior alterations, particularly aggressiveness, social withdrawal and other social behavior alterations.

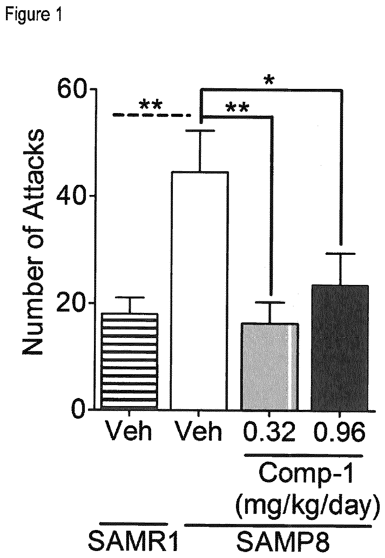

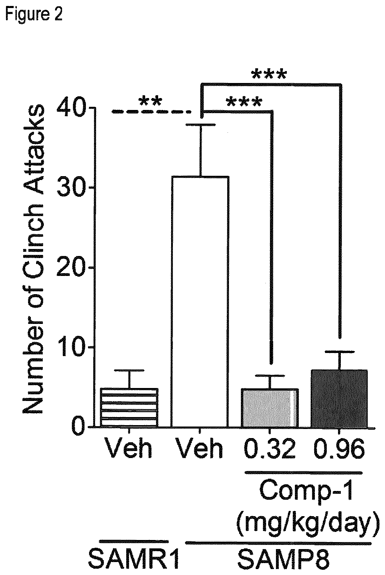

As illustrated in more detail in Example 3 and Figures 1 and 2, KDM1A inhibitors like Compound 1 have been found to be effective in treating aggressiveness. To test the effects of a compound of interest like Compound 1 on aggressiveness, an animal model (for example a rodent model) is selected where vehicle-treated animals are known to develop, or are identified as showing, altered (increased) aggressive behavior compared to control animals, as assessed using a well-established method to measure aggressive behavior, and it is then evaluated whether treatment of said aggressive animals with the compound reduces their aggressive behavior compared to vehicle-treated animals, or even restores aggressive behavior to the (normal) levels of the control animals. Aggressive behavior of animals can be evaluated using any standard method to assess aggressive behavior parameters, such as for example the resident-intruder (RI) test, which can be performed for example as described in more detail in Example 3.1. As an example of a suitable animal model to test aggressiveness, male SAMP8 mice can be used, using male SAMR1 mice as control. As illustrated in Example 3 and in Figures 1 and 2, vehicle-treated male SAMP8 mice exhibit a significantly increased aggressive behavior compared to the control strain SAMR1, as shown by a significantly increased number of total attacks and especially of clinch attacks. Treatment of male SAMP8 mice with a KDM1A inhibitor (particularly Compound 1) drastically reduces their aggressiveness, as illustrated in Figures 1 and 2 by the number of attacks (both total attacks and clinch attacks), which are restored in Compound 1-treated SAMP8 mice to SAMR1 levels. Treatment with the KDM1A inhibitor Compound 1 is thus able to correct the altered aggressive behavior of SAMP8 mice, supporting the use of KDM1A inhibitors to treat aggressiveness and related behavior alterations.

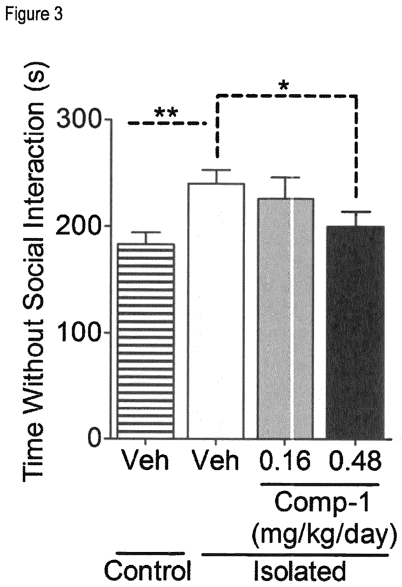

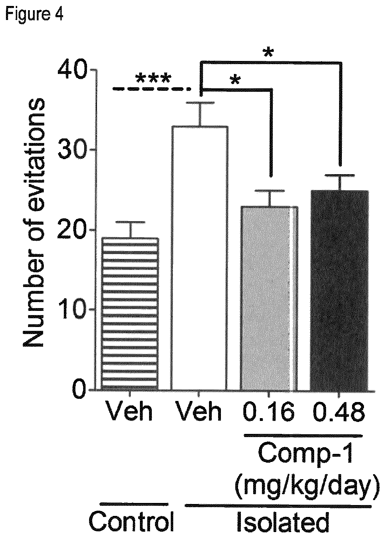

In addition to exerting therapeutic effects on aggressiveness, KDM1A inhibitors like Compound 1 are also useful for treating other behavior alterations like social withdrawal, as illustrated in Example 4 and Figures 3 and 4. While mice are highly territorial, rats are known as a more gregarious species and are thus a particularly suitable species to assess social interaction behavior and particularly social withdrawal. A suitable model to assess social withdrawal is the rat isolation rearing model. In this model, rats are isolated after weaning and deprived of the normal environment that preconditions their social behavior. Isolation in this phase of the development of the rat leads to behavior alterations, particularly a lack of interest for social interactions (social avoidance) in the adult animal, which can be used as a model for human social withdrawal. Social behavior of animals in this model can then be assessed using any standard method for such evaluation known in the art, such as the resident-intruder test. As illustrated in Example 4 and Figures 3 and 4, social avoidance parameters are greatly increased in vehicle-treated isolated rats compared to vehicle-treated non-isolated rats, as reflected by the time without social interaction (Figure 3) and the number of evitations (Figure 4). Treatment with the KDM1A inhibitor Compound 1 greatly reduces social avoidance in isolated rats, as illustrated in Figure 3 by a dose-dependent reduction in the time without social interaction in isolated rats, and in Figure 4 by a reduction in the number of evitations, which was greatly increased in vehicle-treated isolated rats and was restored to normality (i.e. to non-isolated rat levels) by treatment with the KDM1A inhibitor Compound 1. Treatment with a KDM1A inhibitor is able to improve or correct social avoidance, supporting the use of KDM1A inhibitors to treat social withdrawal and related social behavior alterations.

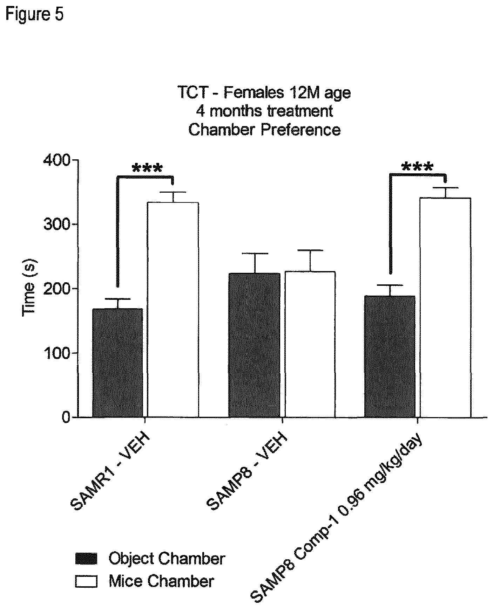

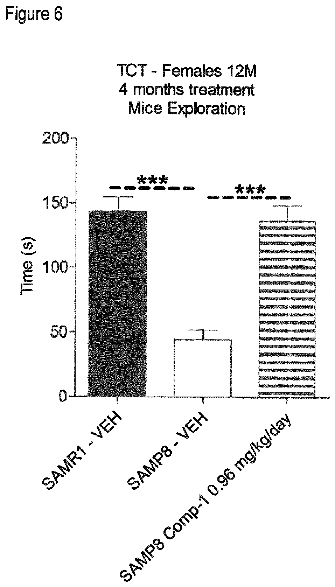

The utility of KDM1A inhibitors for treating social behavior alterations is further illustrated in Example 5 and Figures 5 and 6, using another widely used social behavior test, the Three Chamber Test (TCT). The TCT is a commonly used method to measure social behavior in mice and is useful to assess the effects of a compound to treat social interaction alterations, using animals exhibiting innate or acquired deficits in social behaviour. In the TCT test, as explained in more detail in Example 5, after adaptation to the thee-chamber arena, a mouse is released into the middle chamber and allowed to explore the other compartments. In the adjacent 'mouse' compartment a docile stimulus mouse is situated in a mesh-wire container, while in the other adjacent compartment a similar container is located without stimulus mouse (object compartment). The propensity to approach or avoid the compartment with the stimulus mouse provides a measure of sociability. As illustrated in Example 5 and Figures 5 and 6, treatment with a KDM1A inhibitor like Compound 1 is able to restore social interaction behavior/sociability in subjects exhibiting social interaction alterations. As described in Example 5, contrary to the control strain, SAMR1, female SAMP8 mice show no preference for the "mice" chamber over the "object" chamber and also spend less time exploring the novel mouse, showing thus deficits in social behavior. Treatment of female SAMP8 mice with the KDM1A inhibitor Compound 1 completely restores social interaction behavior/sociability of SAMP8 mice to SAMR1 levels, as shown by restoring both the preference for the socialization chamber (mice chamber) (see Figure 5) and the time spent exploring a novel mice (see Figure 6). Importantly, the therapeutic effects of KDM1A inhibitors like Compound 1 in the treatment of behavior alterations are attainable without producing sedative effects, as illustrated in Examples 3 and 4 using standard assays to measure sedative or anxiolytic effects like the Open Field and the Elevated Plus Maze tests. Sedation is a serious side effect in many drugs currently used to treat behavior alterations. For example, antipsychotic drugs used to treat aggressiveness generally cause strong sedation. KDM1A inhibitors, and particularly Compound 1, are therefore highly advantageous over current treatments in that they can be used to treat behavior alterations, without causing sedative side effects.

As explained above, the present invention relates to a KDM1A inhibitor which is 5-((((1R,2S)-2-(4-(benzyloxy)phenyl)cyclopropyl)amino)methyl)-1,3,4-oxadiazol-2-amine, or a pharmaceutically acceptable salt or solvate thereof, for use in the treatment of a behavior alteration which is social withdrawal or aggressiveness, wherein said social withdrawal or said aggressiveness is associated with a disease.

### KDM1A inhibitors

As used herein, a KDM1A inhibitor is a compound which inhibits KDM1A, particularly human KDM1A.

Both irreversible and reversible KDM1A inhibitors have been reported. Irreversible KDM1A inhibitors exert their inhibitory activity by becoming covalently bound to the FAD cofactor within the KDM1A active site and are generally based on a 2-cyclyl-cyclopropylamino moiety such as a 2-(hetero)arylcyclopropylamino moiety. Reversible inhibitors of KDM1A have also been disclosed.

The KDM1A inhibitor to be used in accordance with the invention is 5-((((1R,2S)-2-(4-(benzyloxy)phenyl)cyclopropyl)amino)methyl)-1,3,4-oxadiazol-2-amine or a pharmaceutically acceptable salt or solvate thereof.

Accordingly, the present invention relates to a KDM1A inhibitor for use in the treatment of a behavior alteration which is social withdrawal or aggressiveness, wherein said social withdrawal or said aggressiveness is associated with a disease, wherein the KDM1A inhibitor is a compound of the following formula: 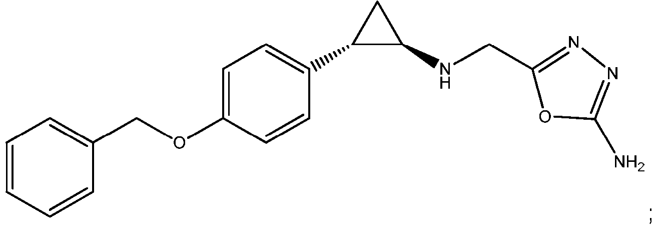 or any pharmaceutically acceptable salt or solvate thereof.

The ability of a compound to inhibit KDM1A can be tested in vitro using any method to determine KDM1A inhibition known in the art, for example the method disclosed in Example 2.

As explained above, the KDM1A inhibitor for use according to the invention is 5-((((1R,2S)-2-(4-(benzyloxy)phenyl)cyclopropyl)amino)methyl)-1,3,4-oxadiazol-2-amine, or a pharmaceutically acceptable salt or solvate thereof.

### Pharmaceutical Formulations

While it is possible that the KDM1A inhibitor, specifically Compound 1, may be administered for use in therapy directly as such, it is typically administered in the form of a pharmaceutical composition, which comprises the compound as active pharmaceutical ingredient together with one or more pharmaceutically acceptable excipients or carriers. Any reference to the KDM1A inhibitor herein includes a reference to the compound as such, i.e. the corresponding compound in non-salt form (e.g., as a free base) or in the form of any pharmaceutically acceptable salt or solvate thereof, as well as a reference to a pharmaceutical composition comprising said compound and one or more pharmaceutically acceptable excipients or carriers.

The KDM1A inhibitor may be administered by any means that accomplish the intended purpose. Examples include administration by the oral, parenteral (including e.g. intravenous, subcutaneous or intracerebral), or topical routes.

For oral delivery, the compound can be incorporated into a formulation that includes pharmaceutically acceptable carriers such as binders (*e.g.,* gelatin, cellulose, gum tragacanth), excipients (*e.g.,* starch, lactose), lubricants (*e.g.,* magnesium stearate, silicon dioxide), disintegrating agents (*e.g.,* alginate, Primogel, and corn starch), and sweetening or flavoring agents (*e.g.,* glucose, sucrose, saccharin, methyl salicylate, and peppermint). The formulation can be orally delivered, e.g., in the form of enclosed gelatin capsules or compressed tablets. Capsules and tablets can be prepared by any conventional techniques. The capsules and tablets can also be coated with various coatings known in the art to modify the flavors, tastes, colors, and shapes of the capsules and tablets. In addition, liquid carriers such as fatty oil can also be included in capsules.

Suitable oral formulations can also be in the form of suspension, syrup, chewing gum, wafer, elixir, and the like. If desired, conventional agents for modifying flavors, tastes, colors, and shapes of the special forms can also be included. In addition, for convenient administration by enteral feeding tube in patients unable to swallow, the active compounds can be dissolved in an acceptable lipophilic vegetable oil vehicle such as olive oil, corn oil and safflower oil.

The compound can also be administered parenterally in the form of solution or suspension, or in lyophilized form capable of conversion into a solution or suspension form before use. In such formulations, diluents or pharmaceutically acceptable carriers such as sterile water and physiological saline buffer can be used. Other conventional solvents, pH buffers, stabilizers, anti-bacteria agents, surfactants, and antioxidants can all be included. For example, useful components include sodium chloride, acetates, citrates or phosphates buffers, glycerin, dextrose, fixed oils, methyl parabens, polyethylene glycol, propylene glycol, sodium bisulfate, benzyl alcohol, ascorbic acid, and the like. The parenteral formulations can be stored in any conventional containers such as vials and ampoules.

For topical administration, the compound can be formulated into lotions, creams, ointments, gels, powders, pastes, sprays, suspensions, drops and aerosols. Thus, one or more thickening agents, humectants, and stabilizing agents can be included in the formulations. Examples of such agents include, but are not limited to, polyethylene glycol, sorbitol, xanthan gum, petrolatum, beeswax, or mineral oil, lanolin, squalene, and the like. A special form of topical administration is delivery by a transdermal patch. Methods for preparing transdermal patches are disclosed, e.g., in Brown, et al. (1988) Ann. Rev. Med. 39:221-229.

Subcutaneous implantation for sustained release of the compound may also be a suitable route of administration. This entails surgical procedures for implanting an active compound in any suitable formulation into a subcutaneous space, *e.g.,* beneath the anterior abdominal wall. See, *e.g.,* Wilson et al. (1984) J. Clin. Psych. 45:242-247. Hydrogels can be used as a carrier for the sustained release of active compounds. Hydrogels are generally known in the art. They are typically made by crosslinking high molecular weight biocompatible polymers into a network, which swells in water to form a gel like material. Preferably, hydrogels are biodegradable or biosorbable. For purposes of this invention, hydrogels made of polyethylene glycols, collagen, or poly(glycolic-co-L-lactic acid) may be useful. See, *e.g.,* Phillips et al. (1984) J. Pharmaceut. Sci., 73: 1718-1720.

The compound can also be conjugated to a water soluble non-immunogenic non-peptidic high molecular weight polymer to form a polymer conjugate. For example, the compound can be covalently linked to polyethylene glycol to form a conjugate. Typically, such a conjugate exhibits improved solubility, stability, and reduced toxicity and immunogenicity. Thus, when administered to a patient, the compound in the conjugate can have a longer half-life in the body, and exhibit better efficacy. See generally, Burnham (1994) Am. J. Hosp. Pharm. 15:210-218. PEGylated proteins are currently being used in protein replacement therapies and for other therapeutic uses. For example, PEGylated interferon (PEG-INTRON A^{®}) is clinically used for treating Hepatitis B. PEGylated adenosine deaminase (ADAGEN^{®}) is being used to treat severe combined immunodeficiency disease (SCIDS). PEGylated L-asparaginase (ONCAPSPAR^{®}) is being used to treat acute lymphoblastic leukemia (ALL). It is preferred that the covalent linkage between the polymer and the active compound and/or the polymer itself is hydrolytically degradable under physiological conditions. Such conjugates known as "prodrugs" can readily release the active compound inside the body. Controlled release of an active compound can also be achieved by incorporating the active ingredient into microcapsules, nanocapsules, or hydrogels generally known in the art. Other pharmaceutically acceptable prodrugs of the compound include, but are not limited to, esters, carbonates, thiocarbonates, N-acyl derivatives, N-acyloxyalkyl derivatives, quaternary derivatives of tertiary amines, N-Mannich bases, Schiff bases, amino acid conjugates, phosphate esters, metal salts and sulfonate esters.

Liposomes can also be used as carriers for the active compound. Liposomes are micelles made of various lipids such as cholesterol, phospholipids, fatty acids, and derivatives thereof. Various modified lipids can also be used. Liposomes can reduce the toxicity of the active compounds, and increase their stability. Methods for preparing liposomal suspensions containing active ingredients therein are generally known in the art. See, e.g., U.S. Patent No. 4,522,811; Prescott, Ed., Methods in Cell Biology, Volume XIV, Academic Press, New York, N. Y. (1976).

The pharmaceutical compositions, like oral and parenteral compositions, can be formulated in unit dosage forms for ease of administration and uniformity of dosage. As used herein, "unit dosage forms" refers to physically discrete units suitable as unitary dosages for administration to subjects, each unit containing a predetermined quantity of active ingredient calculated to produce the desired therapeutic effect, in association with one or more suitable pharmaceutical carriers.

In therapeutic applications, pharmaceutical compositions are to be administered in a manner appropriate to the disease to be treated, as determined by a person skilled in the medical arts. An appropriate dose and suitable duration and frequency of administration will be determined by such factors as the condition of the patient, the type and severity of the disease, the particular form of the active ingredient, the method of administration, among others. In general, an appropriate dose and administration regimen provides the pharmaceutical composition in an amount sufficient to provide therapeutic benefit, for example an improved clinical outcome, such as more frequent complete or partial remissions, or longer disease-free and/or overall survival, or lessening of symptoms severity, or any other objetively identifiable improvement as noted by the clinician. Effective doses may generally be assessed or extrapolated using experimental models like dose-response curves derived from in vitro or animal model test systems like the ones illustrated in the Examples.

The pharmaceutical compositions of the invention can be included in a container, pack or dispenser together with instructions for administration.

KDM1A inhibitors, such as Compound 1, have been found to be orally active and to be effective in the treatment of behavior alterations when administered orally, as also illustrated in Examples 3 and 4. Accordingly, it is preferred that the KDM1A inhibitor (Compound 1) is administered by the oral route for the treatment of a behavior alteration which is social withdrawal or aggressiveness, wherein said social withdrawal or said aggressiveness is associated with a disease.

The present invention also embraces the use of KDM1A inhibitors, in which one or more atoms are replaced by a specific isotope of the corresponding atom. For example, the invention encompasses the use of a KDM1A inhibitor, in which one or more hydrogen atoms (or, e.g., all hydrogen atoms) are replaced by deuterium atoms (i.e., ²H; also referred to as "D"). Accordingly, the invention also embraces KDM1A inhibitors which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in a KDM1A inhibitor can be increased using deuteration techniques known in the art. For example, a KDM1A inhibitor or a reactant or precursor to be used in the synthesis of the KDM1A inhibitor can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that the KDM1A inhibitor to be used in accordance with the present invention is not enriched in deuterium. Accordingly, the presence of naturally occurring hydrogen atoms or ¹H hydrogen atoms in the KDM1A inhibitor is preferred. In general, it is preferred that none of the atoms in the KDM1A inhibitor to be used in accordance with the invention are replaced by specific isotopes. The KDM1A inhibitor or the pharmaceutical composition comprising the KDM1A inhibitor to be used in accordance with the present invention can be administered in monotherapy (e.g., without concomitantly administering any further therapeutic agents, or without concomitantly administering any further therapeutic agents against the same behavior alteration that is to be treated with the KDM1A inhibitor). Accordingly, the KDM1A inhibitor or the pharmaceutical composition comprising the KDM1A inhibitor can be used in the monotherapeutic treatment of a behavior alteration which is social withdrawal associated with a disease or aggressiveness associated with a disease (e.g., without administering any other therapeutic agents against the same behavior alteration until the treatment with the KDM1A inhibitor is terminated). However, the KDM1A inhibitor or the pharmaceutical composition comprising the KDM1A inhibitor can also be administered in combination with one or more further therapeutic agents. If the KDM1A inhibitor is used in combination with a second therapeutic agent active against the same behavior alteration (which is social withdrawal associated with a disease or aggressiveness associated with a disease), the dose of each compound may differ from that when the corresponding compound is used alone, in particular, a lower dose of each compound may be used. The combination of the KDM1A inhibitor with one or more further therapeutic agents may comprise the simultaneous/concomitant administration of the KDM1A inhibitor and the further therapeutic agent(s) (either in a single pharmaceutical formulation or in separate pharmaceutical formulations), or the sequential/separate administration of the KDM1A inhibitor and the further therapeutic agent(s). If administration is sequential, either the KDM1A inhibitor or the one or more further therapeutic agents may be administered first. If administration is simultaneous, the one or more further therapeutic agents may be included in the same pharmaceutical formulation as the KDM1A inhibitor, or they may be administered in one or more different (separate) pharmaceutical formulations (which can be administered via the same or different routes of administration).

### Definitions

Unless defined otherwise, all technical and scientific terms used herein have the same meaning as commonly understood by one of ordinary skill in the art to which this invention pertains.

The following definitions apply throughout the present specification and claims, unless specifically indicated otherwise.

A "patient" or "subject" for the purposes of the present invention includes both humans and other animals, particularly mammals, and other organisms. Thus, the uses of the invention are applicable to both human therapy and veterinary applications. In a preferred aspect the subject or patient is a mammal, and in the most preferred aspect the subject or patient is human.

The term "abnormal" indicates deviating from the normal, average or expected.

The term "inappropriate" indicates that something is in discordance with societal standards and/or expectations. The term "pathological" indicates that something, e.g., a phenomenon or condition, constitutes a disease state or is altered or caused by or related to a disease.

The terms "treatment", "treating" and the like are used herein to generally mean obtaining a desired pharmacological and/or physiological effect. The effect may be prophylactic in terms of completely or partially preventing a disease (herein, a behavior alteration) or symptom thereof and/or may be therapeutic in terms of partially or completely curing or ameliorating a disease (i.e. a behavior alteration) and/or a symptom or adverse effect attributed to the disease or partially or completely halting the progression of a disease and/or a symptom or adverse effect attributed to the disease. The term "treatment" as used herein covers any treatment of a disease (i.e. a behavior alteration) in a patient and includes, without limitation, any one or more of the following: (a) preventing a behavior alteration in a patient which may be predisposed/at risk of developing the behavior alteration; (b) delaying the onset of the behavior alteration; (c) inhibiting the behavior alteration, i.e. arresting, delaying or slowing down its development/progression; or (d) relieving the behavior alteration, i.e. causing (complete or partial) regression, correction or alleviation of the behavior alteration. The present invention specifically and distinctly relates to each one of these forms of treatment.

As used herein, the term "therapeutically effective amount" refers to the amount sufficient to produce a desired biological effect (e.g., a therapeutic effect) in a subject. Accordingly, a therapeutically effective amount of a compound may be an amount which is sufficient to treat a disease, and/or delay the onset or progression of a disease, and/or alleviate one or more symptoms of the disease, when administered to a subject suffering from or susceptible to that disease.

As used herein, a "pharmaceutically acceptable salt" is intended to mean a salt that retains the biological effectiveness of the free acids and/or bases of the specified compound and that is not biologically or otherwise undesirable. A compound may possess a sufficiently acidic, a sufficiently basic, or both functional groups, and accordingly react with any of a number of inorganic or organic bases, and inorganic and organic acids, to form a pharmaceutically acceptable salt. Exemplary pharmaceutically acceptable salts include those salts prepared by reaction of a compound of the invention, specifically Compound 1, with a mineral or organic acid, such as hydrochlorides, hydrobromides, sulfates, pyrosulfates, bisulfates, sulfites, bisulfites, phosphates, monohydrophosphates, dihydrophosphates, metaphosphates, pyrophosphates, chlorides, bromides, iodides, nitrates, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, gamma-hydroxybutyrates, glycollates, tartrates, methane-sulfonates, ethane-sulfonates, propanesulfonates, benzenesulfonates, toluenesulfonates, trifluoromethansulfonates, naphthalene-1-sulfonates, naphthalene-2-sulfonates, mandelates, pyruvates, stearates, ascorbates, or salicylates. When a compound carries an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands such as ammonia, alkylamines, hydroxyalkylamines, lysine, arginine, N-methylglucamine, procaine and the like. Pharmaceutically acceptable salts are well known in the art.

As used herein, a "pharmaceutically acceptable solvate" refers to a complex of variable stoichiometry formed by a solute and a pharmaceutically acceptable solvent such as water, ethanol and the like. A complex with water is known as a hydrate. It is to be understood that the invention encompasses pharmaceutically acceptable solvates of the KDM1A inhibitor in non-salt form and also in the form of a pharmaceutically acceptable salt thereof.

As used herein, a "pharmaceutically acceptable carrier" or "pharmaceutically acceptable excipient" refers to non-API (API refers to Active Pharmaceutical Ingredient) substances such as disintegrators, binders, fillers, and lubricants used in formulating pharmaceutical products. They are generally safe for administering to humans according to established governmental standards, including those promulgated by the United States Food and Drug Administration and/or the European Medicines Agency. Pharmaceutically acceptable carriers or excipients are well known to those skilled in the art.

As used herein, a "small molecule" refers to an organic compound with a molecular weight below 900 daltons, preferably below 500 daltons. The molecular weight is the mass of a molecule and is calculated as the sum of the atomic weights of each constituent element multiplied by the number of atoms of that element in the molecular formula.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of' and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

As used herein, unless explicitly indicated otherwise or contradicted by context, the terms "a", "an" and "the" are used interchangeably with "one or more" and "at least one". Thus, for example, a composition comprising "a" KDM1A inhibitor can be interpreted as referring to a composition comprising "one or more" KDM1A inhibitors.

### EXAMPLES

The following examples illustrate various aspects of the invention. The examples should, of course, be understood to be merely illustrative of only certain embodiments of the invention and not to constitute limitations upon the scope of the invention. Results are also presented and described in the Figures and Figure legends.

### Example 1: Materials

Compound 1 (or Comp. 1) is the compound 5-((((1R,2S)-2-(4-(benzyloxy)phenyl)cyclopropyl)amino)methyl)-1,3,4-oxadiazol-2-amine, also known as (-) 5-((((trans)-2-(4-(benzyloxy)phenyl)cyclopropyl)amino)methyl)-1,3,4-oxadiazol-2-amine, and whose chemical structure is shown below.

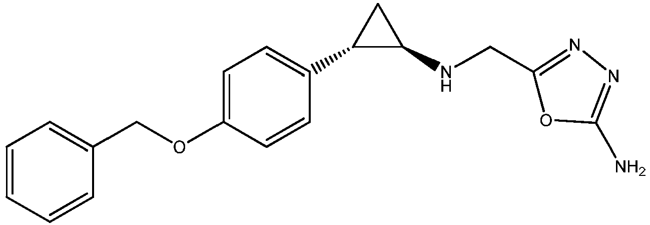

This compound can be obtained as disclosed in WO2012/013728.

### Example 2: In vitro KDM1A inhibition assay

The inhibitory activity of a compound against KDM1A can be determined using the method described below. Human recombinant KDM1A protein (GenBank accession no. NM_015013, amino acids 158-end with N-terminal GST tag, MW: 103 kDa) was used.

Serial 3-fold dilutions of a test compound ranged between 30 µM and 1 nM were pre-incubated for 15 min with human recombinant KDM1A enzyme (BPS Bioscience, Ref. 50100) on ice in the assay buffer (50 mM sodium phosphate pH 7.4). Each concentration of inhibitor was tested in duplicate. The enzymatic reaction was initiated by the addition of dimethyl H3K4 peptide substrate (Anaspec, Ref. 63677), at the appK_{M} of KDM1A. After 30 min of incubation at 37°C Amplex Red reagent and the horseradish peroxidase (HRP) solution were added to detect H₂O₂ formed in the enzymatic reaction, following the recommendations provided by the supplier (Invitrogen). The mix was incubated for 5 min at room temperature in the dark and the conversion of the Amplex Red reagent to the highly fluorescent resorufin was analyzed using an Infinite F200 Tecan fluorescence microplate reader (λexcitation=540 nm, λemission=590 nm). The maximum demethylase activity of KDM1A was obtained in the absence of inhibitor and corrected for background fluorescence in the absence of KDM1A. The IC₅₀ value for each inhibitor was calculated with GraphPad Prism5 Software from a minimum of two independent experiments.

Compound 1 is a KDM1A inhibitor, as shown by a mean IC₅₀ value of 101 ± 40 nM obtained in the KDM1A assay described herein.

### Example 3: Evaluation of the effect of KDM1A inhibitors on aqqressive behavior

The effect of the KDM1A inhibitor Compound 1 on aggressive behavior was evaluated in SAMP8 male mice using the resident-intruder (RI) test. The RI test is a standardized method to measure social behavior, particularly aggressive behavior in a semi-natural setting.

### 3.1 METHOD

SAM mice models were developed from AKR/J mice strain by Kyoto University. SAMP8 litter showed severe senescence and was selected to further propagate and examine these characteristics. SAMR1 litter showed normal aging and was selected as a senescence-resistant strain.

In this study, male SAMP8 animals were treated either with vehicle (n = 5), 0.32 (n = 8) or 0.96 (n = 8) mg/kg/day of Compound 1 for 5 weeks from month 5 of age and then subjected to the RI test. Vehicle (1.8% 2-Hydroxypropyl-β-cyclodextrin, Sigma-Aldrich, Spain) or Compound 1 were administered in drinking water. SAMR1 mice treated with vehicle were included as a control (n = 6). All drugs were administered via drinking water and diluted in vehicle. Drug concentration was calculated weekly in function of body weight and corrected in function of drinking water consumption.

The resident-intruder (RI) test was performed as follows: the test subject (resident) was maintained in its home cage without bedding changes for one week. On the test day, a significantly younger and smaller subject (intruder; 90 days old C57BL6 mouse) was introduced in the resident home cage. The session (20 min) was video-recorded and social interaction (parameters tested: social interaction and rearings) and aggressive behavior (parameters tested: lateral threats, clinch attacks, keep-down behavior, and total attacks as the sum of all three aggressive behavior parameters measured) of the test subject were analyzed by an experimenter blind to the treatment.

Statistical analysis: SAMR1 and SAMP8 vehicle groups were compared by t-Test. Among the SAMP8 groups, different treatments were compared by oneway-ANOVA with Dunnet and SNK post-Hoc analysis.

### 3.2 RESULTS

When the aggressive behavior of SAMP8 mice was compared to that of SAMR1 mice in the RI test, the number of total attacks, and especially clinch attacks, was significantly increased in vehicle-treated SAMP8 relative to vehicle-treated SAMR1 mice, as shown in Figures 1 and 2, indicating SAMP8 male mice exhibit an altered (increased) aggressive behavior as compared to the reference strain SAMR1. Treatment of SAMP8 mice with Compound 1 lowered the number of attacks in SAMP8 mice to SAMR1 levels, as shown in Figures 1 and 2. Compound 1 thus drastically reduced aggressiveness in SAMP8 mice, correcting the altered aggressive behavior of said animals.

Vehicle-treated SAMP8 animals did not show significant differences relative to vehicle-treated SAMR1 mice in time spent in social interaction. No significant differences were observed in the number of rearings in vehicle-treated SAMP8 versus SAMR1 mice, nor did treatment with Compound 1 affect this readout in SAMP8 mice.

### 3.3 EFFECTS OF KDM1A INHIBITORS ON AGGRESSIVE BEHAVIOR ARE NOT DUE TO SEDATION

To confirm that the effects of Compound 1 on aggressive behavior of SAMP8 male mice were due to a direct effect of the compound on the aggressiveness of the animals and not caused by a potential sedative effect of the compound, the effects of Compound 1 on the anxiety and locomotor activity of male SAMP8 mice were studied using the open field (OF) and the elevated plus maze (EPM) tests, as described below.

### 3.3.1 METHOD

Animals (n = 8/group) were treated from month 5 of age with vehicle, 0.32 or 0.96 mg/kg/day Compound 1, and by month 7 of treatment they were sequentially submitted to the OF and EPM test at one week intervals.

Vehicle (1.8% 2-Hydroxypropyl-β-cyclodextrin, Sigma-Aldrich, Spain) or KDM1A inhibitor (Compound 1) were administered in drinking water. SAMR1 mice treated with vehicle were included as a control (n = 8). All drugs were administered via drinking water and diluted in vehicle. Drug concentration was calculated weekly in function of body weight and corrected in function of drinking water consumption.

The OF and EPM tests were performed as follows:
Open Field (OF): A 50 × 50-cm white plastic arena with 25-cm-high walls was used to analyze spontaneous exploratory behavior. The floor of the apparatus was divided into 25 equal squares. The movements of each animal were video recorded during 5 min. Locomotor activity was analyzed by video-tracking over the captured images using SMART^{®} (v3.0, PanLab, SLU, Spain).

Elevated Plus Maze (EPM): The EPM consisted of four arms at right angles to each other connected to a central square and maintained elevated 50 cm above the floor. Two of the opposite arms had high walls (enclosed arms, 30 x 5 x 15 cm), whereas the other two were open arms (30 x 5 x 0 cm). The animal was placed facing a closed arm, and its movements were video-recorded for 5 min and analyzed by video-tracking using SMART^{®} (v3.0, PanLab, SLU, Spain).

Statistical analysis: SAMR1 and SAMP8 vehicle groups were compared by t-Test. Among the SAMP8 groups, different treatments were compared by oneway-ANOVA with Dunnet and SNK post-Hoc analysis.

### 3.3.2 RESULTS

No significant differences were observed between vehicle-treated SAMR1 and vehicle-treated SAMP8 mice in the OF test, nor did treatment with Compound 1 significantly affect locomotor activity or the time spent in the center zone. SAMP8 mice spent a significantly increased time in the open arms of the EPM relative to SAMR1 mice, but this behavior was not significantly modified by Compound 1. Therefore, Compound 1 did not have anxiolytic or sedative activity in SAMP8 mice.

Summarized, the data and results obtained in Example 3 show that the KDM1A inhibitor Compound 1, administered at doses that are well-tolerated by mice for long-term treatment, drastically reduced aggressiveness but it did not work as a sedative or anxiolytic drug in SAMP8 mice. Example 3 thus supports the finding that KDM1A inhibitors, particularly Compound 1, can be used for the treatment of behavior alterations such as aggressiveness, without causing sedation.

### Example 4: Evaluation of the effect of KDM1A inhibitors on social withdrawal

While mice are highly territorial, rats are known as a more gregarious species. To further characterise the therapeutic effects of KDM1A inhibitors like Compound 1 for the treatment of behavior alterations, the effect of Compound 1 on social withdrawal, another type of behavior alteration, was assessed in rats using the rat isolation rearing model.

In this model, rats are isolated after weaning on postnatal day 21 (PND21) and deprived of the normal environment that preconditions their social behavior. Isolation in this phase of the development of the rat may lead to behavior alterations, particularly a lack of interest for social interactions, which can be used as a model for human social withdrawal.

### 4.1 METHOD

Right after the weaning (post natal day 21-23), 48 Sprague-Dawley male rats were divided in two groups: Control (Non Isolated; n=12), maintained 3-4 animals per cage; Isolated (n=36), 1 animal per cage. Starting on postnatal day (PND) 61, adult isolated male rats were treated with vehicle, Compound 1 at 0.16 mg/kg/day or Compound 1 at 0.48 mg/kg/day (n = 12/group) for 5 weeks. Control (non-isolated) animals were treated with vehicle. The administration route was drinking water and the vehicle 1.8% 2-hydroxypropyl-β-cyclodextrin. Drug concentration was adjusted weekly by body weight and water consumption. During the last week of treatment, all animals were tested in the RI (PND94) to evaluate social behavior and, on a different day, in the EPM test (PND87-88) to evaluate anxiety behavior.

The resident-intruder (RI) test was performed following a similar protocol to that described for mice in Example 3 above, as follows: Briefly, the test subject (resident) was maintained in its home cage without bedding changes for one week. On the test day (PND94), a significantly younger and smaller subject (intruder; 50 days old Sprague-Dawley rat) was introduced in the resident home cage. The session was video-recorded during 15 min, and social interaction (parameters measured: active and passive social interaction, avoidance and time without social interaction) and aggressive behavior of the test subject were analyzed by an experimenter blind to the treatment.

The elevated plus maze (EPM) test was perfomed following a similar protocol to that described for mice in Example 3 above, as follows: The EPM consisted of four arms at right angles to each other connected to a central square and maintained elevated 50 cm above the floor. Two of the opposite arms had high walls (enclosed arms, 46.5 x 12 x 42 cm), whereas the other two were open arms (46.5 x 12 x 0.3 cm). The animal was placed facing a closed arm, and its movements were video-recorded for 5 min and analyzed by video-tracking using SMART^{®} (v2.5.21, PanLab, SLU, Spain).

Statistical analysis: Non Isolated and Isolated vehicle groups were compared by t-Test. Among the Isolated groups, different drug treatments were compared by oneway-ANOVA with Dunnet and SNK post-Hoc analysis.

### 4.2 RESULTS

The RI test as performed did not reveal aggressive behavior in the rats nor did isolation significantly affect active or passive social interaction. However, the social avoidance parameters were greatly increased in vehicle-treated isolated rats compared to vehicle-treated non-isolated rats, as assessed by the time without social interaction (Figure 3) and the number of evitations (Figure 4). The time without social interaction in isolated rats was dose dependently reduced by treatment with Compound 1 and the number of evitations, which was greatly increased in isolated rats, was restored to normality (i.e. to non-isolated rat levels) by treatment with Compound 1 (see Figure 3 and 4).

In the EPM test, no differences were observed between vehicle-treated isolated vs vehicle-treated non-isolated rats. Compound 1 did not produce any significant effect on anxiety or locomotor activity in the rats as assessed in the EPM, indicating that the beneficial effects produced by Compound 1 on the social avoidance parameters measured in the RI test are not due to a sedative effect of the drug.

Summarized, the data and results obtained in Example 4 show that the KDM1A inhibitor Compound 1, administered at doses that are well-tolerated by rats for long-term treatment, corrected behavior alterations, particularly social avoidance, in rats isolated after weaning without causing sedation. Example 4 thus further supports the finding that KDM1A inhibitors, particularly Compound 1, can be used for the (non-sedative) treatment of behavior alterations, including social withdrawal.

### Example 5: Evaluation of the effect of KDM1A inhibitors using the Three Chamber Test (TCT) in mice

The KDM1A inhibitor Compound 1 was further tested in an additional animal model for social behavior alterations, the three chamber test (TCT). The TCT is a commonly used method to measure social behavior in mice and can be used to assess the effects of a compound to treat social interaction alterations using animals exhibiting innate or acquired deficits in social behaviour.

In this test, after adaptation to the thee-chamber arena, a mouse is released into the middle chamber and allowed to explore the other compartments. In the adjacent 'mouse' compartment a docile stimulus mouse is situated in a mesh-wire container, while in the other adjacent compartment a similar container is located without stimulus mouse (object compartment). The propensity to approach or avoid the compartment with the stimulus mouse provides a measure of social interaction behavior/sociability. Wild type mice prefer social interaction and spend more time in the mouse compartment in comparison to the object compartment.

### 5.1 METHOD

In this study, 8-month old female SAMP8 mice were treated either with vehicle (n= 9) or 0.96 mg/kg/day of Compound 1 (n= 12) for 4 months and then subjected to the TCT. Vehicle (1.8% 2-Hydroxypropyl-β-cyclodextrin, Sigma-Aldrich, Spain) or Compound 1 (diluted in Vehicle) were administered in drinking water. SAMR1 mice treated with vehicle were included as a control (n=11).. Drug concentration was calculated weekly in function of body weight and corrected in function of drinking water consumption.

The TCT was performed in a Plexiglas transparent box with three identical consecutive chambers (15 x 15 x 20 cm) with two identical small metallic cages placed in both lateral chambers. Adjacent chambers were communicated and animals were free to move from one to another. The test subject (SAMR1 or SAMP8 female mice) was allowed to explore the apparatus for 5 minutes. This habituation time was monitored to avoid animals showing preference for one of the chambers. Then, a novel female mouse was introduced in one of the metallic cages (Mice Chamber) while the other cage remained empty (Object Chamber). The time spent in each chamber and the time of direct exploration of the novel mice was measured during a total observation time of 10 minutes.

Statistical analysis: The t test was used to assess the significance of the difference in exploration of novel mice for the SAMP8 relative to SAMR1 mice and for the Compound 1 treated relative to Vehicle treated SAMP8 mice. 2 way ANOVA was used to assess the significance of the chamber preference. ***: p < 0.001.

### 5.2 RESULTS

The results obtained in this test are shown in Figures 5 and 6. As shown in Figure 5, vehicle-treated SAMR1 female mice spent more time in the Mice chamber in comparison to the Object chamber. Contrary to the control SAMR1 animals, vehicle-treated female SAMP8 mice showed no preference for the Mice chamber over the Object chamber (see Figure 5) and also spent less time exploring the novel mouse compared to SAMR1 mice (see Figure 6), showing thus deficits in social behavior. Treatment of female SAMP8 mice with the KDM1A inhibitor Compound 1 restored both the preference for the socialization chamber (mice chamber) (Figure 5) and the time spent exploring a novel mouse (Figure 6) of SAMP8 mice to SAMR1 levels. Compound 1 thus completely corrected the social interaction alterations/lack of sociability of SAMP8 mice.

The results obtained in Example 5 further show that KDM1A inhibitors like Compound 1 can be used for the (non-sedative) treatment of social behavior alterations.

## Claims

1. A KDM1A inhibitor which is 5-((((1R,2S)-2-(4-(benzyloxy)phenyl)cyclopropyl)amino)methyl)-1,3,4-oxadiazol-2-amine, or a pharmaceutically acceptable salt or solvate thereof, for use in the treatment of a behavior alteration, wherein the behavior alteration is social withdrawal or aggressiveness, wherein said social withdrawal or said aggressiveness is associated with a disease.

2. A pharmaceutical composition for use in the treatment of a behavior alteration, wherein the behavior alteration is social withdrawal or aggressiveness, wherein said social withdrawal or said aggressiveness is associated with a disease, and wherein the pharmaceutical composition comprises a KDM1A inhibitor which is 5-((((1R,2S)-2-(4-(benzyloxy)phenyl)cyclopropyl)amino)methyl)-1,3,4-oxadiazol-2-amine, or a pharmaceutically acceptable salt or solvate thereof, and one or more pharmaceutically acceptable excipients or carriers.

3. The compound for use according to claim 1 or the pharmaceutical composition for use according to claim 2, wherein the behavior alteration is social withdrawal associated with a disease.

4. The compound for use according to claim 1 or the pharmaceutical composition for use according to claim 2, wherein the behavior alteration is aggressiveness associated with a disease.

5. The compound for use according to any one of claims 1, 3 or 4 or the pharmaceutical composition for use according to any one of claims 2 to 4, wherein the disease is a CNS disease.

6. The compound for use according to claim 3 or the pharmaceutical composition for use according to claim 3, wherein the disease is autism spectrum disorder, schizophrenia, a mood disorder or post-traumatic stress disorder.

7. The compound for use according to claim 3 or the pharmaceutical composition for use according to claim 3, wherein the disease is autism spectrum disorder.

8. The compound for use according to claim 3 or the pharmaceutical composition for use according to claim 3, wherein the disease is schizophrenia.

9. The compound for use according to claim 3 or the pharmaceutical composition for use according to claim 3, wherein the disease is post-traumatic stress disorder.

10. The compound for use according to claim 4 or the pharmaceutical composition for use according to claim 4, wherein the disease is borderline personality disorder, autism spectrum disorder, adult attention deficit hyperactivity disorder, Alzheimer's disease, Parkinson's disease, schizophrenia or a stress-related disorder.

11. The compound for use according to claim 4 or the pharmaceutical composition for use according to claim 4, wherein the disease is borderline personality disorder.

12. The compound for use according to claim 4 or the pharmaceutical composition for use according to claim 4, wherein the disease is autism spectrum disorder.

13. The compound for use according to claim 4 or the pharmaceutical composition for use according to claim 4, wherein the disease is Alzheimer's disease.

14. The compound for use according to claim 4 or the pharmaceutical composition for use according to claim 4, wherein the disease is schizophrenia.

15. The compound for use according to claim 4 or the pharmaceutical composition for use according to claim 4, wherein the disease is a stress-related disorder.

16. The compound for use according to claim 15 or the pharmaceutical composition for use according to claim 15, wherein the stress-related disorder is post-traumatic stress disorder.

17. The compound for use according to any one of claims 1 or 3 to 16 or the pharmaceutical composition for use according to any one of claims 2 to 16, wherein the patient to be treated is a human.

18. The compound for use according to any one of claims 1 or 3 to 17 or the pharmaceutical composition for use according to any one of claims 2 to 17, wherein the KDM1A inhibitor is 5-((((1R,2S)-2-(4-(benzyloxy)phenyl)cyclopropyl)amino)methyl)-1,3,4-oxadiazol-2-amine.

19. The compound for use according to any one of claims 1 or 3 to 18 or the pharmaceutical composition for use according to any one of claims 2 to 18, wherein the KDM1A inhibitor or the pharmaceutical composition is administered orally.

## Patentansprüche

1. KDM1A-Inhibitor, der 5-((((1R,2S)-2-(4-(Benzyloxy)phenyl)cyclopropyl)amino)methyl)-1,3,4-oxadiazol-2-amin oder ein pharmazeutisch verträgliches Salz oder Solvat davon ist, zur Verwendung bei der Behandlung einer Verhaltensveränderung, wobei die Verhaltensveränderung sozialer Rückzug oder Aggressivität ist, wobei der soziale Rückzug oder die Aggressivität mit einer Erkrankung in Zusammenhang steht.

2. Arzneimittel zur Verwendung bei der Behandlung einer Verhaltensveränderung, wobei die Verhaltensveränderung sozialer Rückzug oder Aggressivität ist, wobei der soziale Rückzug oder die Aggressivität mit einer Erkrankung in Zusammenhang steht, wobei das Arzneimittel einen KDM1A-Inhibitor, der 5-((((1R,2S)-2-(4-(Benzyloxy)phenyl)cyclopropyl)amino)methyl)-1,3,4-oxadiazol-2-amin oder ein pharmazeutisch verträgliches Salz oder Solvat davon ist, und ein oder mehrere pharmazeutisch verträgliche Exzipienten oder Träger umfasst.

3. Verbindung zur Verwendung nach Anspruch 1 oder Arzneimittel zur Verwendung nach Anspruch 2, wobei die Verhaltensveränderung mit einer Erkrankung in Zusammenhang stehender sozialer Rückzug ist.

4. Verbindung zur Verwendung nach Anspruch 1 oder Arzneimittel zur Verwendung nach Anspruch 2, wobei die Verhaltensveränderung mit einer Erkrankung in Zusammenhang stehende Aggressivität ist.

5. Verbindung zur Verwendung nach einem der Ansprüche 1, 3 oder 4 oder Arzneimittel zur Verwendung nach einem der Ansprüche 2 bis 4, wobei die Erkrankung eine Erkrankung des Zentralen Nervensystems (ZNS) ist.

6. Verbindung zur Verwendung nach Anspruch 3 oder Arzneimittel zur Verwendung nach Anspruch 3, wobei die Erkrankung Autismus-Spektrum-Störung, Schizophrenie, eine affektive Störung oder posttraumatische Belastungsstörung ist.

7. Verbindung zur Verwendung nach Anspruch 3 oder Arzneimittel zur Verwendung nach Anspruch 3, wobei die Erkrankung Autismus-Spektrum-Störung ist.

8. Verbindung zur Verwendung nach Anspruch 3 oder Arzneimittel zur Verwendung nach Anspruch 3, wobei die Erkrankung Schizophrenie ist.

9. Verbindung zur Verwendung nach Anspruch 3 oder Arzneimittel zur Verwendung nach Anspruch 3, wobei die Erkrankung posttraumatische Belastungsstörung ist.

10. Verbindung zur Verwendung nach Anspruch 4 oder Arzneimittel zur Verwendung nach Anspruch 4, wobei die Erkrankung Borderline-Persönlichkeitsstörung, Autismus-Spektrum-Störung, Aufmerksamkeitsdefizit-/Hyperaktivitätsstörung bei Erwachsenen, Alzheimer-Krankheit, Morbus Parkinson, Schizophrenie oder eine stressbedingte Störung ist.

11. Verbindung zur Verwendung nach Anspruch 4 oder Arzneimittel zur Verwendung nach Anspruch 4, wobei die Erkrankung Borderline-Persönlichkeitsstörung ist.

12. Verbindung zur Verwendung nach Anspruch 4 oder Arzneimittel zur Verwendung nach Anspruch 4, wobei die Erkrankung Autismus-Spektrum-Störung ist.

13. Verbindung zur Verwendung nach Anspruch 4 oder Arzneimittel zur Verwendung nach Anspruch 4, wobei die Erkrankung Alzheimer-Krankheit ist.

14. Verbindung zur Verwendung nach Anspruch 4 oder Arzneimittel zur Verwendung nach Anspruch 4, wobei die Erkrankung Schizophrenie ist.

15. Verbindung zur Verwendung nach Anspruch 4 oder Arzneimittel zur Verwendung nach Anspruch 4, wobei die Erkrankung eine stressbedingte Störung ist.

16. Verbindung zur Verwendung nach Anspruch 15 oder Arzneimittel zur Verwendung nach Anspruch 15, wobei die stressbedingte Störung posttraumatische Belastungsstörung ist.

17. Verbindung zur Verwendung nach einem der Ansprüche 1 oder 3 bis 16 oder Arzneimittel zur Verwendung nach einem der Ansprüche 2 bis 16, wobei der zu behandelnde Patient ein Mensch ist.

18. Verbindung zur Verwendung nach einem der Ansprüche 1 oder 3 bis 17 oder Arzneimittel zur Verwendung nach einem der Ansprüche 2 bis 17, wobei der KDM1A-Inhibitor 5-((((1R,2S)-2-(4-(Benzyloxy)phenyl)cyclopropyl)amino)methyl)-1,3,4-oxadiazol-2-amin ist.

19. Verbindung zur Verwendung nach einem der Ansprüche 1 oder 3 bis 18 oder Arzneimittel zur Verwendung nach einem der Ansprüche 2 bis 18, wobei der KDM1A-Inhibitor oder das Arzneimittel oral verabreicht wird.

## Revendications

1. Inhibiteur de KDMIA qui est la 5-((((1R,2S)-2-(4-(benzyloxy)phényl)cyclopropyl)amino)méthyl)-1,3,4-oxadiazol-2-amine, ou un solvate ou sel pharmaceutiquement acceptable de celle-ci, pour une utilisation dans le traitement d'une altération du comportement, dans lequel l'altération du comportement est un retrait social ou une agressivité, dans lequel ledit retrait social ou ladite agressivité est associé à une maladie.

2. Composition pharmaceutique pour une utilisation dans le traitement d'une altération du comportement, dans laquelle l'altération du comportement est un retrait social ou une agressivité, dans laquelle ledit retrait social ou ladite agressivité est associé à une maladie, et laquelle composition pharmaceutique comprend un inhibiteur de KDMIA qui est la 5-((((1R,2S)-2-(4-(benzyloxy)phényl)cyclopropyl)amino)méthyl)-1,3,4-oxadiazol-2-amine, ou un solvate ou sel pharmaceutiquement acceptable de celle-ci, et un ou plusieurs véhicules ou excipients pharmaceutiquement acceptables.

3. Composé pour une utilisation selon la revendication 1 ou composition pharmaceutique pour une utilisation selon la revendication 2, dans lequel l'altération du comportement est un retrait social associé à une maladie.

4. Composé pour une utilisation selon la revendication 1 ou composition pharmaceutique pour une utilisation selon la revendication 2, dans lequel l'altération du comportement est une agressivité associée à une maladie.

5. Composé pour une utilisation selon l'une quelconque des revendications 1, 3 et 4 ou composition pharmaceutique pour une utilisation selon l'une quelconque des revendications 2 à 4, dans lequel la maladie est une maladie du SNC.

6. Composé pour une utilisation selon la revendication 3 ou composition pharmaceutique pour une utilisation selon la revendication 3, dans lequel la maladie est un trouble du spectre autistique, une schizophrénie, un trouble de l'humeur ou un trouble de l'état de stress post-traumatique.

7. Composé pour une utilisation selon la revendication 3 ou composition pharmaceutique pour une utilisation selon la revendication 3, dans lequel la maladie est un trouble du spectre autistique.

8. Composé pour une utilisation selon la revendication 3 ou composition pharmaceutique pour une utilisation selon la revendication 3, dans lequel la maladie est une schizophrénie.

9. Composé pour une utilisation selon la revendication 3 ou composition pharmaceutique pour une utilisation selon la revendication 3, dans lequel la maladie est un trouble de l'état de stress post-traumatique.

10. Composé pour une utilisation selon la revendication 4 ou composition pharmaceutique pour une utilisation selon la revendication 4, dans lequel la maladie est un trouble de la personnalité limite, un trouble du spectre autistique, un trouble déficitaire de l'attention avec hyperactivité chez l'adulte, une maladie d'Alzheimer, une maladie de Parkinson, une schizophrénie ou un trouble lié au stress.

11. Composé pour une utilisation selon la revendication 4 ou composition pharmaceutique pour une utilisation selon la revendication 4, dans lequel la maladie est un trouble de la personnalité limite.

12. Composé pour une utilisation selon la revendication 4 ou composition pharmaceutique pour une utilisation selon la revendication 4, dans lequel la maladie est un trouble du spectre autistique.

13. Composé pour une utilisation selon la revendication 4 ou composition pharmaceutique pour une utilisation selon la revendication 4, dans lequel la maladie est une maladie d'Alzheimer.

14. Composé pour une utilisation selon la revendication 4 ou composition pharmaceutique pour une utilisation selon la revendication 4, dans lequel la maladie est une schizophrénie.

15. Composé pour une utilisation selon la revendication 4 ou composition pharmaceutique pour une utilisation selon la revendication 4, dans lequel la maladie est un trouble lié au stress.

16. Composé pour une utilisation selon la revendication 15 ou composition pharmaceutique pour une utilisation selon la revendication 15, dans lequel la maladie liée au stress est un trouble de l'état de stress post-traumatique.

17. Composé pour une utilisation selon l'une quelconque des revendications 1 ou 3 à 16 ou composition pharmaceutique pour une utilisation selon l'une quelconque des revendications 2 à 16, dans lequel le patient devant être traité est un humain.

18. Composé pour une utilisation selon l'une quelconque des revendications 1 ou 3 à 17 ou composition pharmaceutique pour une utilisation selon l'une quelconque des revendications 2 à 17, dans lequel l'inhibiteur de KDM1A est la 5-((((1R,2S)-2-(4-(benzyloxy)phényl)cyclopropyl)amino)méthyl)-1,3,4-oxadiazol-2-amine.

19. Composé pour une utilisation selon l'une quelconque des revendications 1 ou 3 à 18 ou composition pharmaceutique pour une utilisation selon l'une quelconque des revendications 2 à 18, dans lequel l'inhibiteur de KDMIA ou la composition pharmaceutique est administré par voie orale.
